# EUROPEAN PATENT SPECIFICATION

(11) **EP 3 651 857 B1**
(45) Date of publication and mention of the grant of the patent: **12.06.2024**
(21) Application number: 18769554.9
(22) Date of filing: 20.08.2018
(51) Int. Cl.: A61Q 11/00, A61K 8/22, A61K 8/24, A61K 8/81, A61K 8/89

(54) **ORAL CARE WHITENING COMPOSITIONS**
BLEICHZUSAMMENSETZUNGEN ZUR MUNDPFLEGE
COMPOSITIONS DE BLANCHIMENT POUR SOINS BUCCO-DENTAIRES

(30) Priority: 18.08.2017 US 201762547215 P
(43) Date of publication of application: 20.05.2020
(73) Proprietor: Colgate-Palmolive Company, New York, NY 10022 (US)
(72) Inventor: EVANS, Lauren, 08904 Highland Park, NJ (US); CHOPRA, Suman, Monroe, New Jersey 08831 (US); GALIYARA, Tanaz, East Brunswick, New Jersey 08816 (US)
(74) Representative: Wibbelmann, Jobst
(86) International application number: PCT/US2018/047023
(87) International publication number: WO 2019/036696

(56) References cited:
- EP-A1- 1 781 380
- WO-A1-2017/095405
- US-A1- 2006 062 744
- US-A1- 2007 071 695
- US-A1- 2007 071 696
- US-B2- 6 682 721
- US-B2- 6 689 344
- US-B2- 9 155 688

## Description

### BACKGROUND

Products that are presently available to whiten teeth include a variety of different ingredients, but the primary active ingredient is a peroxide source. These products typically contain substantial amounts of whitening agents, for example, a peroxide source in an amount equivalent to about 10% hydrogen peroxide. However, there is a need for compositions having lower concentrations of a peroxide source that are still effective in whitening teeth and provide good microrobustness. Embodiments of the present invention are directed, in part, to this end.

US 2007/071696 A1 discloses a dual phase whitening oral care composition. US 2007/071695 A1 relates to an anhydrous single phase whitening dentifrice for whitening tooth surfaces.

### SUMMARY

The present invention concerns a substantially anhydrous oral care composition having a water content of less than 5% by weight, comprising:
a peroxide source comprising cPVP-hydrogen peroxide complex in an amount to provide from 0.01% to 5.5% of hydrogen peroxide by weight of the composition;
a hydrophobic component comprising a silicone polymer;
sodium tripolyphosphate (STPP); in an amount of from 0.05 to 3% by weight of the composition; wherein the composition optionally comprises a dental surface adhesion agent.

Further embodiments are set forth in the attached claims. Thus, some embodiments of the present invention comprise a substantially anhydrous or non-aqueous oral care composition comprising: a peroxide source comprising hydrogen peroxide bound to cross-linked polyvinylpyrrolidone (cPVP) in the amount effective to provide from 0.01% to 5.5% of hydrogen peroxide, by weight of the composition; a hydrophobic component comprising a silicone polymer; sodium tripolyphosphate; and optionally a dental surface adhesion enhancing agent, e.g., comprising cPVP, for example, wherein the composition when applied to the teeth is sufficiently viscous to form an adherent, continuous layer on a dental surface and deliver an effective amount of said peroxide source to a tooth surface. In some embodiments, the composition is free of polyethylene thickening agent.

Further areas of applicability of the present invention will become apparent from the detailed description and examples provided hereinafter. It should be understood that the detailed description and specific examples, while providing specific embodiments of the invention, are intended for illustration only.

### DETAILED DESCRIPTION

In some embodiments, the composition of the present invention is a viscous liquid, preferably a gel, which maintains its consistency during storage enabling the product to be painted on the tooth surface with a soft applicator pen or brush.

In some embodiments, the composition of the present invention provides a stable vehicle that prevents the decomposition of the peroxide whitening agent during storage and before use.

The peroxide agent of the present invention comprises at least cross-linked polyvinylpyrrolidone-hydrogen peroxide complex (cPVP-H₂O₂) in an amount to provide from 0.01 to 5.5% hydrogen peroxide by weight of the composition. In some embodiments, the composition comprises cPVP-H₂O₂ in an amount to provide from 0.01 to 1% hydrogen peroxide by weight of the composition, for example, from 0.01 to 0.5%, or from 0.05 to 0.25%, or from 0.1 to 0.2%, or about 0.1% hydrogen peroxide by weight of the composition. In some embodiments, the composition comprises cPVP-H₂O₂ in an amount to provide from 3.5 to 5.5%, or from 4.0 to 5.0%, or from 4.25 to 4.75%, or about 4.5% hydrogen peroxide by weight of the composition. cPVP-peroxide complex consists of hydrogen peroxide molecules intimately bound to the polyvinylpyrrolidone backbone. cPVP-peroxide is available commercially as a dry powder for incorporation into whitening and oxidizing compositions. cPVP-peroxide complex typically consists of from 15 to 30 wt% hydrogen peroxide, e.g., 15 to 25% or 18-20%, or about 18%.

The compositions of the present invention have a water content of the composition of less than 5% by weight, or less than 4%, or less than 3% or less than 2% or less than 1%, or less than 0.5%, and range down to 0% (no measurable water).

Once applied on a tooth surface, the saliva on the tooth enamel surface to which the composition is applied will either dissolve or disintegrate the peroxide containing matrix resulting in a rapid decomposition of the peroxide, and thereby provide an effective concentration of the peroxide source at the tooth surface, despite its relatively low concentration in the composition. Surprisingly, this concentration is capable of delivering an acceptable level of tooth whitening.

It has been unexpectedly found that the inclusion of sodium tripolyphosphate (STPP) in the composition, in an amount from 0.05 to 3% by weight of the composition, results in improved resistance to microbial growth and contamination (microrobustness) and improved whitening effectiveness. Optionally the concentration of sodium tripolyphosphate is from 0.1 to 2%, or 0.1 to 0.5%, or 1.5 to 2%, or 0.1% or 2%. Compositions of the prior art typically contain an antimicrobial or preservative agent to inhibit the microbial growth that can cause spoilage of toothpastes and other dentifrice compositions. Examples of such agents include quaternary ammonium salts such as cetylpyridinium chloride (CPC), and zinc ion sources, such as zinc oxide. The present inventors have unexpectedly found that the inclusion of appropriate concentrations of STPP, as provided herein, helps contribute to microrobustness in these compositions sufficiently that no added antimicrobial agent or preservative is needed. Without being bound by theory, it is believed that STPP chelates metal ions that are important in microbial metabolism, thus inhibiting microbial growth. Thus, in some embodiments, the compositions do not comprise antimicrobial agents or preservative agents (e.g., CPC and zinc oxide). STPP also provides important benefits in prevent stain formation on the teeth. Moreover, CPC has been shown to contribute to stain formation in some cases, due its cationic character, and the inclusion of STPP and the absence of CPC further contributes to the prevention of teeth staining.

The whitening compositions of the present invention are portable viscous liquid or gel tooth whiteners that can be applied to the teeth as a coated layer conveniently painted onto the tooth enamel surface. Upon application to the teeth, the applied whitening composition forms an adherent layer of peroxide containing product that has the capacity to release the peroxide whitening agent over an extended period of time, e.g., from about 5 minutes to about 12 hours. The applied layer adheres to the tooth surface whereby the released peroxide source then whitens the teeth to which the composition is applied.

The viscosity of a composition of the invention is greater than about 1,000 centipoise (cPs) and less than about 900,000 cPs, in a more specific embodiment greater than about 10,000 cP and less than about 100,000 cPs, in a more specific embodiment greater than 50,000 cPs and less than 900,000 cPs, and in an even more specific embodiment from between about 200,000 cPs to about 600,000 cPs.

In some embodiments, the present invention comprises a hydrophobic component, carrier or base material that comprises a silicone polymer. The term "hydrophobic" or "water-insoluble" as applied to polymers and as employed herein refers to an organic polymer which is substantially non-aqueous having a water solubility of less than one gram per 100 grams of water at 25°C. Any such silicone polymers that are compatible with the whitening agents described herein, and which can produce a tooth whitening composition having a desired viscosity can be used.

The present invention comprises a hydrophobic component comprising at least one hydrophobic polymer, for example, "siloxane" polymers, which are also generally known in the art as "silicone" polymers. In certain embodiments, the hydrophobic polymers that comprise the hydrophobic material are those in which a whitening agent can be dispersed and are well known in the art. Many such silicone polymers are commercially available. In various embodiments, a preferred silicone-based hydrophobic polymer is a polyorganosiloxane, in particular polydimethylsiloxane (e.g., dimethicone).

In some embodiments, the siloxane polymers that can function as part of the hydrophobic component are in the form of a fluid. Polysiloxane fluids useful herein include those with a viscosity, at 25° C, of about 1 milliPascal-sec (mPa-s) to about 1000 mPa-s, or about 2 mPa-s to about 500 mPa-s, or about 20 mPa-s to about 400 mPa-s. Polysiloxane fluids for use herein can be linear or cyclic, and can be substituted with a wide variety of substituents. In certain embodiments, substituents include methyl, ethyl and phenyl substituents. Suitable polysiloxane fluids include linear polysiloxane polymers such as dimethicone and other low viscosity analogues of the polysiloxane materials, in certain embodiments having a viscosity, at 25° C., of 200 mPa-s or less and cyclomethicone, and other cyclic siloxanes having for example a viscosity, at 25° C., of 200 mPa-s or less. Other fluids include polysiloxane polyether copolymers and hydroxy terminated polydimethyl-siloxane fluid (e.g., Dow Corning ST-DIMETHICONOL.TM. 40, Dow Corning SGM 36, SGM3). Commercial examples of materials that are suitable for use herein include DC200 series fluids marketed by Dow-Corning Corporation and the AK Fluid series marketed by Wacker-Chemie GmbH, Munchen, Germany. High molecular silicone resins with a polysiloxane blend may also be used including powdered trimethylsiloxysilicate, for example, Dow Corning 593 fluid, Wacker Belsil TMS 803. Another suitable silicone fluid from Dow Corning is Q7-9210.

In some embodiments, at least part of the hydrophobic component is a silicone pressure sensitive adhesive (PSA). Such PSAs can be produced by condensing a silicone resin and an organosiloxane such as a polydiorganosiloxane. Such hydrophobic polymers are an elastomeric, tacky material, adhesion of which to dental enamel surfaces can be varied by altering the ratio of silicone resin to polydiorganosiloxane in the copolymer molecule. Such polymers are pressure sensitive hydrophobic polymers specifically designed for pharmaceutical use and are permeable to many drug compounds and find application for the transdermal application of various compounds. In some embodiments, the silicone polymers are the copolymer product of mixing a silanol terminated polydiorganosiloxane such as polydimethyl siloxane with a silanol-containing silicone resin whereby the silanol groups of the polydiorganosiloxane undergo a condensation reaction with the silanol groups of the silicone resin so that the polydiorganosiloxane is lightly crosslinked by the silicone resin (that is, the polydiorganosiloxane chains are bonded together through the resin molecules to give chain branching and entanglement and/or a small amount of network character) to form the silicone hydrophobic polymers. A catalyst, for example, an alkaline material, such as ammonia, ammonium hydroxide or ammonium carbonate, can be mixed with the silanol-terminated polydiorganosiloxane and the silicone resin to promote this crosslinking reaction. By copolymerizing the silicone resin with the silanol terminated polydiorganosiloxane, there results a polymer with self-adhering properties and the cohesive properties of a soft elastomer matrix characteristic of pressure sensitive polymers being distinguished from the hard, non-elastomeric properties of other silicone resins. In one embodiment, hydrophobic polymers used in the carrier are available from the Dow-Corning Company under the brand name BIO-PSA. The modification of a ratio of silicone resin to polydiorganosiloxane modifies the tackiness of the polymer. This ratio can be in the range of about 70:30 to about 50:50. For example, the BIO PSA silicone sold by Dow-Corning is available in three silicone resin to silicone polymer ratios namely, 65/35 (low tack), 60/40 (medium tack), 55/45 (high tack). Such a polyorganosiloxane PSA is available dissolved in either ethyl acetate solvent or dimethicone. Modifying the silicone resin to polydiorganosiloxane ratio of the PSA will modify the tackiness of the PSA. For example, the BIO PSA silicone adhesive sold by Dow-Corning is available in three silicone resin to silicone polymer ratios namely, 65/35 (low tack), 60/40 (medium tack), 55/45 (high tack) dissolved in either ethyl acetate solvent or dimethicone. A suitable silicone PSA is Silicone Adhesive 8-7016, commercially available from Dow Corning.

In some embodiments, the silicone adhesive is a pressure sensitive silicone adhesive. In some embodiments, the pressure sensitive silicone adhesive is a copolymer prepared by condensing a silicone resin with a polydiorganosiloxane. In some embodiments, the polydiorganosiloxane is polydimethylsiloxane. In some embodiments, the silicone resin is a silanol-containing silicone resin.

In some embodiments, the hydrophobic component further comprises a natural or synthetic hydrocarbon, such as mineral oil, petrolatum, or liquid paraffin, e.g., white petrolatum. In some embodiments, the composition comprises from 10 to 30% of a natural or synthetic hydrocarbon, e.g., from 15 to 25%, by weight of the composition.

In some embodiments, the hydrophobic component is present at a concentration of from 20 to 80%, by weight of the composition, optionally, from 40 to 80%, or from 60 to 80%, or from 70 to 80%, or from 70 to 75%, by weight of the composition. In some embodiments, the hydrophobic component comprises one or more silicone polymers, and said silicone polymer are present in a net concentration of 30 to 70%, or 40 to 60%, or 45 to 55%, or about 45%, or about 50%, or about 55%, by weight of the composition. In some embodiments, the hydrophobic component consists of one or more silicone polymers combined with a natural or synthetic hydrocarbon. In some embodiments, the hydrophobic component does not comprise polyethylene (including polyethylene/mineral oil blends, also known as Plastigels).

The present inventors have discovered that particular amounts of a dental surface adhesion enhancing agent not only provides greater retention of the composition to the tooth surface, but also enhances the stability of the peroxide source and helps to maximize delivery of an effective concentration of the peroxide source at the target site. Dental surface adhesion agents are polar organic polymers which, due to their polarity, tend to adhere electrostatically to the surface of the tooth. Dental surface adhesion agents may be any polar organic polymers, including anionic polymers, cationic polymers, and neutral polar polymers. Suitable anionic polymers include polyacrylate polymers and copolymers of methyl vinyl ether with maleic acid or anhydride. Suitable neutral polar polymers include polyvinylpyrrolidone (PVP), including either linear PVP or cross-linked PVP (cPVP), or a mixture thereof. In some embodiments, the total amount of dental surface adhesion agent in the composition ranges from 10% to 40%, by weight of the composition. Such concentration of dental surface adhesion agent includes the cPVP present in the cPVP-hydrogen peroxide complex as described herein. In some embodiments, the only dental surface adhesion agent present is PVP, e.g., linear PVP, cPVP or a mixture thereof (including the cPVP present in the cPVP-peroxide complex).

In some embodiments, the total amount of dental surface adhesion agent, e.g., PVP (including linear PVP, cPVP or mixtures thereof, including the cPVP in cPVP-H₂O₂) is from 10% to 30%, or from 15% to 25%, or from 20% to 25%, or about 20%, or about 22% or about 24%, by weight of the composition. In some embodiments, the only source of dental surface adhesion agent is the cPVP present in the cPVP-hydrogen peroxide complex. In other embodiments, substantially all of the dental surface adhesion agent (e.g., from 95 to 99% thereof) is provided by added dental surface adhesion agent (e.g., added cPVP, linear PVP, or a mixture thereof).

In some embodiments, adhesiveness may be measured using standard adhesion tests known in the art, for example, the adhesive test disclosed in U.S. Pat. No. 6,613,812 to Bui. In certain embodiments, the adhesiveness between a tooth and a film formed from a composition of the present invention can be from about at least 500 pounds per square inch (PSI), at least 1,000 PSI, at least 2,000 PSI, or greater.

In some embodiments, the compositions of the present invention optionally comprise a tartar control or anti-calculus agent, in addition to the aforementioned sodium tripolyphosphate. Such agent are also useful as stain-prevention agents in the present compositions. Such agents include salts of any of these agents, for example their alkali metal and ammonium salts: phosphates and polyphosphates (for example pyrophosphates), polyaminopropanesulfonic acid (AMPS), polyolefin sulfonates, polyolefin phosphates, diphosphonates such as azacycloalkane-2,2-diphosphonates (e.g., azacycloheptane-2,2-diphosphonic acid), N-methyl azacyclopentane-2,3-diphosphonic acid, ethane-1-hydroxy-1,1-diphosphonic acid (EHDP) and ethane-1-amino-1,1-diphosphonate, phosphonoalkane carboxylic acids and. Useful inorganic phosphate and polyphosphate salts include monobasic, dibasic and tribasic sodium phosphates, sodium tripolyphosphate, tetrapolyphosphate, mono-, di-, tri- and tetrasodium pyrophosphates, sodium trimetaphosphate, sodium hexametaphosphate (SHMP) and mixtures thereof. In a particular embodiment, SHMP is used. The amount of such agent optionally present is from about 0.1% to about 10%, in another embodiment from about 2% to about 9%, and in another embodiment from about 5% to about 8%, or about 7%, by weight, of the composition.

In some embodiments, the compositions of the present invention comprise a flavoring agent. Suitable flavoring agents include, but are not limited to, essential oils as well as various flavoring aldehydes, esters, alcohols, and similar materials. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Of these, the most commonly employed are the oils of peppermint, spearmint and wintergreen. The flavoring agent is incorporated in the whitening compositions of the present invention at a concentration of about 0.01 to about 2% by weight and preferably about 0.1 to about 0.5% by weight.

In some embodiments, the composition further comprises an additional whitening agent selected from: hydrogen peroxide; urea peroxide, sodium percarbonate, sodium perborate; and a combination of two or more thereof.

The composition according to the invention can be maintained on the surface of a tooth for from about 1 minute to about 8 hours. In some embodiments, the composition is maintained on the surface of a tooth for from about 5 minutes to about 4 hours. In some embodiments, the composition can be maintained on the surface of a tooth for from about 10 minutes to about 120 minutes. In some embodiments, the composition is maintained on the surface of a tooth for from about 15 minutes to about 60 minutes. In some embodiments, the composition is maintained on the surface of a tooth for from about 20 minutes to about 45 minutes. In some embodiments, the composition can be maintained on the surface of a tooth for about 30 minutes.

Typically, the compositions are applied directly to the teeth, e.g., by painting the teeth for a time sufficient to effect whitening. The compositions of the present invention can be used in a regimen for whitening teeth and can be used in combination with a whitening toothpaste and a whitening mouthwash to further enhance the whitening results.

Some embodiments provide a method wherein the applicator is a pen and the pen is stored within an oral care implement. In some embodiments, the pen is removed from the oral care implement prior to application of the composition to the tooth. In some embodiments, the composition is applied to the tooth after brushing. In some embodiments, the composition is applied to the tooth after brushing with the oral care implement.

As used herein, "whitening" refers to a change in visual appearance of a tooth, preferably such that the tooth has a brighter shade. Increase in whiteness of a dental surface can be observed visually, for example with the aid of color comparison charts or gauges, or measured by colorimetry, using any suitable instrument such as a Minolta Chromameter, e.g., model CR-400 (Minolta Corp., Ramsey, N.J.). The instrument can be programmed, for example, to measure Hunter Lab values or L*a*b* values according to the standard established by the International Committee of Illumination (CIE). The L*a*b* system provides a numerical representation of three-dimensional color space where L* represents a lightness axis, a* represents a red-green axis and b* represents a yellow-blue axis. The L* and b* axes are typically of greatest applicability to measurement of tooth whiteness. Increase in whiteness can be computed from differences in L*, a* and b* values before and after treatment, or between untreated and treated surfaces.

As used herein, "tooth" or "teeth" refers to natural mammalian teeth, dentures, dental plates, fillings, caps, crowns, bridges, dental implants, and any other hard surfaced dental prosthesis either permanently or temporarily fixed within the oral cavity.

As used throughout, ranges are used as shorthand for describing each and every value that is within the range. Any value within the range can be selected as the terminus of the range. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

Embodiments of the present invention are further described in the following examples. The examples are merely illustrative

### EXAMPLES

### Example 1

Table 1 (below) provides the formulations for exemplary compositions according to the present disclosure.

**Table 1**

| | **Formula I** | **Formula II** |
|---|---|---|
| **Ingredient** | **Weight %** | |
| Silicone adhesive | 20-40 (e.g. 30) | 20-40 (e.g. 30) |
| Silicone fluid | 10-30 (e.g. 18) | 15-35 (e.g. 25) |
| PVP-H₂O₂ complex (e.g., 18 wt. % H₂O₂) | 0.05-5.5 (e.g. 0.55) | 20-30 (e.g. 25) |
| STPP | 0.05-3 (e.g. 2) | 0.05-3 (e.g. 0.1) |
| Hydrophobic polymer (e.g. white petrolatum) | 10-30 (e.g. 25) | 15-25 (e.g. 18) |
| Adhesion agent (e.g. PVP or cPVP) | 10-30 (e.g. 22) | -- |
| Flavor, sweetener | 0.5-3 (e.g. 1.1) | 0.5-3 (e.g. 1.1) |
| Silica | 0-5 (e.g. 1.0) | 0-5 (e.g. 0.5 or 1.0) |

### Example 2

Stain prevention efficacy of a composition according to Composition 1, shown in Table 2 below, is determined using an established stain prevention protocol. The composition according to Composition 1 is compared to a clinically proven, commercially available whitening mouthwash, Composition A (positive control), against distilled water (negative control) and against a peroxide and STPP-free placebo of Composition 1 (Comparative Composition B). Composition A contains 2.7% tetrapotassium pyrophosphate, 0.9% tetrasodium pyrophosphate, 0.34% zinc citrate, and 0.1% sodium fluoride in an aqueous base comprising water, glycerin, sorbitol and propylene glycol. Comparative Composition B differs from Composition 1 essentially only in the removal of sodium tripolyphosphate and hydrogen peroxide.

**Table 2**

| | **Composition 1** | **Comparative Composition B** |
|---|---|---|
| **Ingredient** | **Weight %** | **Weight %** |
| Silicone adhesive DC 8-7016 | 30% | 30% |
| Silicone fluid Q7-9120 | 16% | 16% |
| PVP-H₂O₂ complex (18 wt. % H₂O₂) | 25% | 0% |
| PVP | 0% | 27% |
| STPP | 2% | 0% |
| White petrolatum | 25% | 25% |
| Flavor, sweetener | 1.1% | 1.1% |
| Silica | 1.0% | 1.0% |

The protocol is intended to mimic a consumer's typical daily routine: use of the whitening product, followed by several exposures to staining agents, followed by a second use of the whitening product. Briefly, bovine teeth are mounted in methacrylic resin blocks. Baseline optical measurements are taken using a Spectroshade Micro spectrophotometer (Medical High Technology), and whiteness index (WIO) values are calculated. Bovine teeth samples are divided into three groups with balanced mean WIO values. For the distilled water group, the bovine blocks are immersed in distilled water for 15 minutes at 37 °C. For the Composition 1 group, the teeth are covered by the composition and submerged in 20 mL of artificial saliva for 15 minutes at 37 °C. For the Composition A group, the bovine blocks are submerged in the mouthwash product for 15 minutes at 37 °C. This constitutes the first treatment. A staining broth is then prepared by combining filtered red wine, fresh black tea, and freshly prepared instant coffee in equal amounts with vigorous stirring. After rising off any material from the first treatment, the bovine blocks are submerged in the staining broth for 20 minutes at room temperature. After draining the broth, the bovine blocks are submerged in artificial saliva for 20 minutes at room temperature. The staining broth/saliva cycle is repeated four times. A second treatment is then applied as was done for the first treatment. Spectrophotometer measurements are then repeated and L*, a* and b* values are determined and used to calculate the whiteness index (WIO) score. The change in WIO from baseline to end of study is calculated (ΔWIO).

The results are shown in Table 3 below. The larger the value of ΔWIO, the closer the tooth color is to white at the end of the study. These results demonstrate that Composition 1 is highly effective at preventing stain formation, comparable in efficacy to the clinically proven whitening mouthwash Comparative Composition A. The comparison between Composition 1 and Comparative Composition B demonstrates that the whitening efficacy of Composition 1 is attributable to the presence of the PVP-peroxide complex and STPP.

**Table 3**

| | **ΔWIO** |
|---|---|
| **Distilled water** | -72.6 |
| **Comparative Composition B** | -48.0 |
| **Composition 1** | -22.1 |
| **Comparative Composition A** | -19.4 |

### Example 3

Compositions according to Formulations I and II are prepared and compared in whitening efficacy to prior art formulations comprising a polyethylene backbone. The assay used is a simple in-vitro whitening assay, similar to that described for Example 2. Briefly, mounted bovine teeth are submerged in deionized water, then removed and coated for 2 minutes with the test composition. The teeth are then rinsed three times with deionized water. Each such treatment cycle is repeated 28 times. Spectrophotometer measurements are taken before the first treatment (baseline) and after the seventh, fourteenth, twenty-first and/or twenty-eighth treatments. L*, a* and b* values are measured and a whiteness score, W* is calculated. W* is a measure of overall color change relative to pure white. The applicable formula is W* = ((a*)² + (b*)² + (L*-100)²)^{½}. ΔW is the difference in whiteness score between the test measure and the baseline score. The compositions tested are shown in Table 4.

**Table 4**

| | **Composition 2** | **Comparative 2-C** | **Composition 3** | **Comparative 3-C** |
|---|---|---|---|---|
| **Ingredient** | **Weight %** | | | |
| | 4.5 wt.% hydrogen peroxide | | 0.1 wt.% hydrogen peroxide | |
| Silicone adhesive DC 8-7016 | 30% | 30% | 30% | 30% |
| Silicone fluid Q7-9120 | 25% | 14% | 18% | 16% |
| PVP-H₂O₂ complex (18 wt. % H₂O₂) | 25% | 25% | 0.55% | 0.55% |
| cPVP adhesion agent | -- | -- | 22% | 18% |
| STPP | 0.10% | -- | 2.0% | -- |
| White petrolatum | 18% | -- | 25% | -- |
| Flavor, sweetener | 1.1% | 0.9% | 1.1% | 0.9% |
| Silica | 0.5% | -- | 1.0% | -- |
| Plastigel 5 (mineral oil/polyethylene blend) | -- | 30% | -- | 35% |

The results of the whitening efficacy study are shown in Table 5 (below). The results demonstrate that the substitution of white petrolatum for Plastigel 5, and the addition of STPP, results in equivalent whitening efficacy to the prior art compositions.

**Table 5**

| | **ΔW Whitening at Treatment:** | | | |
|---|---|---|---|---|
| | **7** | **14** | **21** | **28** |
| **Composition 2** | -8.1 | -10.7 | -11.2 | |
| **Comparative 2-C** | -7.4 | -9.9 | -11.2 | |
| **Composition 3** | -1.0 | -1.6 | -1.6 | -2.2 |
| **Comparative 3-C** | -0.9 | -1.5 | -1.6 | -2.3 |

### Example 4

A composition, according to formula I, for whitening gel in pen containing polyphosphate, 4.5% hydrogen peroxide and 0.1% STPP in non-PE base, is compared to a Placebo comparative composition hydrogen peroxide-free and STPP-free (0% HP and 0% STPP). The authors of this invention have discovered a technology (sodium tripolyphosphate) that when added in a Whitening formula containing 4.5% hydrogen peroxide in Non-PE base, provides a stain prevention benefit. Attempts to boost the whitening performance of the hydrogen peroxide formulations with other different technologies have not yet been successful. However, this new formulation surprisingly provides a large stain prevention benefit and this novel approach can be easily used for whitening teeth at-home.

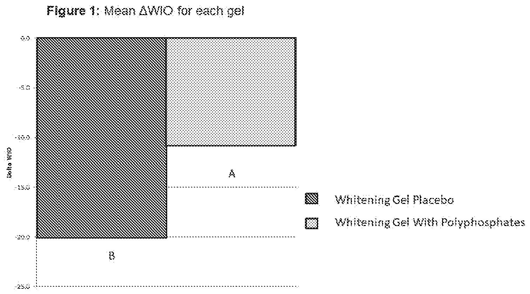

The methodology used to prove the stain prevention efficacy is same as used and described in detail in the Example 2 and involves cycles of product treatment followed by staining with a tea/coffee/wine solution. The calculations for measurements of ΔWIO are performed as previously described in the Example 2. The two different treatments groups are analyzed using statistical Tukey method and results of Tukey analysis are shown in the third column of the Table 6 below. Mean ΔWIO for each gel are shown in the Table 6 below and Figure 1. Samples which do not share same alphabet in the Table 6, third column, are statistically significantly (p < 0.05) different.

The results suggest that the group treated with gel containing 4.5 %HP plus 0.1% STTP had whiter teeth, because they had lower ΔWIO values, when compared to the comparative placebo group treated with gel lacking the HP and STPP. The two groups are statistically significantly different from each other as determined by Tukey method.

**Table 6**

| **Product** | **Mean (Δ WIO)** | **Grouping Information Using Tukey Method** |
|---|---|---|
| Whitening Gel Placebo (0% HP + 0 % STPP) | -20.1195 | B |
| Whitening Gel with Polyphoshate (4.5% HP + 0.1% STPP) | -10.7900 | A |

### Example 5

A composition according to Formula I is prepared and compared in a microrobustness assay against compositions having the antimicrobial agents CPC and zinc oxide, respectively. The compositions tested are shown in Table 7 below.

**Table 7**

| | **Composition 4** | **Comparative D** | **Comparative E** |
|---|---|---|---|
| **Ingredient** | **Weight %** | | |
| Silicone adhesive DC 8-7016 | 30% | 30% | 30% |
| Silicone fluid Q7-9120 | 18% | 18% | 18% |
| PVP-H₂O₂ complex (18 wt. % H₂O₂) | 0.55% | 0.55% | 0.55% |
| cPVP adhesion agent | 22% | 24% | 21% |
| **STPP** | **2.0%** | **--** | **--** |
| White petrolatum | 25% | 25% | 25% |
| Flavor, sweetener | 1.1% | 0.9% | 1.1% |
| Abrasive silica | 1.0% | 1.0% | 1.0% |
| **CPC** | **--** | **0.1%** | **--** |
| **Zinc Oxide** | **--** | **--** | **1.0%** |

The Micro Robustness Index (MRI) of each of these compositions is measured. The Micro Robustness Index is used as a quantitative measure of a composition's ability to withstand microbial challenge. The MRI value results from a challenge test assessing the antimicrobial efficacy of a composition against a pool of common oral microorganisms, such as *Burkholderia*, *Enterobacter*, *Escherichia*, *Klebsiella*, *Serratia*, *Providencia*, *Pseudomonas*, and *Staphylococcus* species. Samples are challenged 3 times at 30-minute intervals with an inoculum of 10⁷ bacteria from the above listed pool. After 4, 6 and 24 hours, aliquots are tested to measure the log reduction of bacteria. Using this data, the area under the curve (AUC) is calculated and then converted into the MRI score; the higher the MRI, the greater the microrobustness of the tested composition.

The results are shown in Table 8 below. It is demonstrated that the use of either 1% zinc oxide or 0.1% CPC provides approximately equivalent antimicrobial robustness, but unexpectedly, it is found that the use of 2% STPP produces improved antimicrobial robustness compared to either CPC or zinc oxide alone.

**Table 8**

| | **MRI** |
|---|---|
| **Composition 4** | 0.77 |
| **Comparative D** | 0.70 |
| **Comparative E** | 0.69 |

## Claims

1. A substantially anhydrous oral care composition having a water content of less than 5% by weight, comprising:
a peroxide source comprising cPVP-hydrogen peroxide complex in an amount to provide from 0.01% to 5.5% of hydrogen peroxide by weight of the composition;
a hydrophobic component comprising a silicone polymer;
sodium tripolyphosphate (STPP); in an amount of from 0.05 to 3% by weight of the composition;
wherein the composition optionally comprises a dental surface adhesion agent.

2. The composition of claim 1, wherein the composition comprises said dental surface adhesion enhancing agent, e.g., polyvinylpyrrolidone (PVP) or cross-linked PVP.

3. The composition of any foregoing claim, wherein the cPVP-hydrogen peroxide complex is present in an amount to provide from 0.01 to 1% hydrogen peroxide, by weight of the composition.

4. The composition of claim 1 or claim 2, wherein the cPVP-hydrogen peroxide complex is present in an amount to provide from 3.5 to 5.5% hydrogen peroxide, by weight of the composition.

5. The composition of any foregoing claim, wherein the composition comprises less than 4% water by weight of the composition.

6. The composition of any foregoing claim, wherein the composition comprises the hydrophobic component in an amount of from 20 to 80%, by weight of the composition.

7. The composition of any preceding claim, wherein the composition does not comprise polyethylene thickener.

8. The composition of any foregoing claim, wherein the dental surface adhesion agent is selected from an anionic polymer, a cationic polymer, and neutral polar polymer, e.g., a polyacrylate polymer, a copolymer of methyl vinyl ether with maleic acid or anhydride, and linear PVP or cross-linked PVP, or any mixtures thereof.

9. The composition of any foregoing claim, wherein the total amount of dental surface adhesion agent is from 10% to 40%, by weight of the composition.

10. The composition of any foregoing claim, wherein the hydrophobic component further comprises a natural or synthetic hydrocarbon, e.g., mineral oil, petrolatum, or liquid paraffin, e.g., white petrolatum, or
wherein the composition does not comprise an antimicrobial or preservative agent, e.g., does not comprise cetylpyridinium chloride, or zinc oxide, or both.

11. The composition of any preceding claim, wherein the composition comprises:
| | |
|---|---|
| | |
| **Ingredient** | **Weight %** |
|---|---|
| Silicone adhesive | 20-40 (e.g. 30) |
| Silicone fluid | 10-40 (e.g. 25) |
| PVP-H₂O₂ complex (e.g., 18 wt. % H₂O₂) | 15-35 (e.g. 25) |
| STPP | 0.05-3 (e.g. 0.1) |
| Hydrophobic polymer (e.g. white petrolatum) | 10-30 (e.g. 18) |
| Flavor, sweetener | 0.5-3 (e.g. 1.1) |
| Silica | 0-5 (e.g. 0.5 or 1.0) |
or
| | |
|---|---|
| | |
| **Ingredient** | **Weight %** |
|---|---|
| Silicone adhesive | 20-40 (e.g. 30) |
| Silicone fluid | 10-30 (e.g. 18) |
| PVP-H₂O₂ complex (e.g., 18 wt. % H₂O₂) | 0.05-5.5 (e.g. 0.55) |
| STPP | 0.05-3 (e.g. 2) |
| Hydrophobic polymer (e.g. white petrolatum) | 10-30 (e.g. 25) |
| Adhesion agent (e.g. PVP or cPVP) | 10-30 (e.g. 22) |
| Flavor, sweetener | 0.5-3 (e.g. 1.1) |
| Abrasive silica | 0-5 (e.g. 1.0) |

12. A composition according to any one of the foregoing claims for use in a method for whitening a tooth comprising applying said composition to a tooth of a mammal.

13. The composition for use of claim 12, wherein the composition is applied using a pen.

14. The composition for use of claim 12 or 13, wherein the pen is stored within an oral care implement.

## Patentansprüche

1. Im wesentlichen wasserfreie Mundpflegezusammensetzung mit einem Wassergehalt von weniger als 5 Gew.-%, umfassend:
eine Peroxidquelle, die einen cPVP-Wasserstoffperoxidkomplex in einer Menge umfasst, die 0,01 % bis 5,5 % Wasserstoffperoxid, bezogen auf das Gewicht der Zusammensetzung, liefert; eine hydrophobe Komponente, die ein Silikonpolymer umfasst;
Natriumtripolyphosphat (STPP); in einer Menge von 0,05 bis 3 Gew.-% der Zusammensetzung; wobei die Zusammensetzung gegebenenfalls ein dentales Oberflächenadhäsionsmittel umfasst.

2. Zusammensetzung nach Anspruch 1, wobei die Zusammensetzung das dentale Oberflächenadhäsionsverbesserungsmittel, z. B. Polyvinylpyrrolidon (PVP) oder vernetztes PVP, umfasst.

3. Zusammensetzung nach einem der vorstehenden Ansprüche, wobei der cPVP-Wasserstoffperoxid-Komplex in einer Menge vorhanden ist, die 0,01 bis 1 % Wasserstoffperoxid, bezogen auf 20 Gew.-% der Zusammensetzung, ergibt.

4. Zusammensetzung nach Anspruch 1 oder Anspruch 2, wobei der cPVP-Wasserstoffperoxid-Komplex in einer Menge vorhanden ist, die 3,5 bis 5,5 % Wasserstoffperoxid, bezogen auf das Gewicht der Zusammensetzung, ergibt.

5. Zusammensetzung nach einem der vorstehenden Ansprüche, wobei die Zusammensetzung weniger als 4 % Wasser, bezogen auf das Gewicht der Zusammensetzung, enthält.

6. Zusammensetzung nach einem der vorstehenden Ansprüche, wobei die Zusammensetzung die hydrophobe Komponente in einer Menge von 20 bis 80 Gew.-% der Zusammensetzung enthält.

7. Zusammensetzung nach einem der vorstehenden Ansprüche, wobei die Zusammensetzung kein Polyethylen-Verdickungsmittel enthält.

8. Zusammensetzung nach einem der vorstehenden Ansprüche, wobei das dentale Oberflächenadhäsionsmittel aus einem anionischen Polymer, einem kationischen Polymer und einem neutralen polaren Polymer, z.B. einem Polyacrylatpolymer, einem Copolymer von Methylvinylether mit Maleinsäure oder -anhydrid und linearem PVP oder vernetztem PVP oder beliebigen Mischungen davon ausgewählt ist.

9. Zusammensetzung nach einem der vorstehenden Ansprüche, wobei die Gesamtmenge des dentalen Oberflächenhaftungsmittels 10 bis 40 Gew.-% der Zusammensetzung beträgt.

10. Zusammensetzung nach einem der vorstehenden Ansprüche, wobei die hydrophobe Komponente weiterhin einen natürlichen oder synthetischen Kohlenwasserstoff, z.B. Mineralöl, Petrolatum oder flüssiges Paraffin, z.B. weißes Petrolatum, enthält,
oder wobei die Zusammensetzung kein antimikrobielles oder konservierendes Mittel enthält, z. B. kein Cetylpyridiniumchlorid oder Zinkoxid oder beides.

11. Zusammensetzung nach einem der vorstehenden Ansprüche, wobei die Zusammensetzung umfasst:
| | |
|---|---|
| | |
| **Bestandteil** | **Gewichts- %** |
|---|---|
| Silikonadhäsionsmittel | 20-40 (z.B. 30) |
| Silikonflüssigkeit | 10-40 (z.B. 25) |
| PVP-H₂0₂-Komplex (z.B. 18 Gew.-%. H₂0₂) | 15-35 (z.B. 25) |
| STPP | 0,05-3 (z.B. 0,1) |
| Hydrophobes Polymer (z. B. weiße Vaseline) | 10-30 (z.B. 18) |
| Aroma, Süßstoff | 0,5-3 (z.B. 1,1) |
| Kieselerde | 0-5 (z.B. 0,5 oder 1.0) |
oder
| | |
|---|---|
| | |
| **Bestandteil** | **Gewichts- %** |
|---|---|
| Silikonadhäsionsmittel | 20-40 (z.B. 30) |
| Silikonflüssigkeit | 10-30 (z. B. 18) |
| PVP- H₂0₂-Komplex (z.B. 18 Gew.-% H₂0₂) | 0,05-5,5 (z.B. 0.55) |
| STPP | 0,05-3 (z.B. 2) |
| Hydrophobes Polymer (z. B. weiße Vaseline) | 10-30 (z.B. 25) |
| Haftvermittler (z. B. PVP oder CPVP) | 10-30 (z.B. 22) |
| Aroma, Süßstoff | 0,5-3 (z. B. 1,1) |
| Abrasive Kieselsäure | 0-5 (z.B. 1,0) |

12. Zusammensetzung nach einem der vorstehenden Ansprüche zur Verwendung in einem Verfahren zum Aufhellen eines Zahns, das das Auftragen der Zusammensetzung auf einen Zahn eines Säugetiers umfasst.

13. Zusammensetzung zur Verwendung nach Anspruch 12, wobei die Zusammensetzung mit einem Stift aufgetragen wird.

14. Zusammensetzung zur Verwendung nach Anspruch 12 oder 13, wobei der Stift in einem Mundpflegegerät aufbewahrt wird.

## Revendications

1. Composition de soins bucco-dentaires sensiblement anhydre ayant une teneur en eau inférieure à 5 % en poids, comprenant :
une source de peroxyde comprenant un complexe cPVP-peroxyde d'hydrogène en une quantité pour fournir de 0,01 % à 5,5 % de peroxyde d'hydrogène en poids de la composition ;
un constituant hydrophobe comprenant un polymère de silicone ;
du tripolyphosphate de sodium (STPP), en une quantité de 0,05 à 3 % en poids de la composition ;
dans laquelle la composition comprend éventuellement un agent d'adhérence à la surface dentaire.

2. Composition selon la revendication 1, dans laquelle la composition comprend ledit agent améliorant l'adhérence à la surface dentaire, par exemple la polyvinylpyrrolidone (PVP) ou la PVP réticulée.

3. Composition selon l'une quelconque des revendications précédentes, dans laquelle le complexe cPVP-peroxyde d'hydrogène est présent en une quantité pour fournir de 0,01 à 1 % de peroxyde d'hydrogène, en poids de la composition.

4. Composition selon la revendication 1 ou la revendication 2, dans laquelle le complexe cPVP-peroxyde d'hydrogène est présent en une quantité pour fournir de 3,5 à 5,5 % de peroxyde d'hydrogène, en poids de la composition.

5. Composition selon l'une quelconque des revendications précédentes, dans laquelle la composition comprend moins de 4 % d'eau en poids de la composition.

6. Composition selon l'une quelconque des revendications précédentes, dans laquelle la composition comprend le constituant hydrophobe en une quantité de 20 à 80 % en poids de la composition.

7. Composition selon l'une quelconque des revendications précédentes, dans laquelle la composition ne comprend pas d'épaississant polyéthylène.

8. Composition selon l'une quelconque des revendications précédentes, dans laquelle l'agent d'adhérence à la surface dentaire est choisi parmi un polymère anionique, un polymère cationique et un polymère polaire neutre, par exemple un polymère polyacrylate, un copolymère d'éther méthylvinylique avec de l'acide ou de l'anhydride maléique, et une PVP linéaire ou une PVP réticulée, ou tout mélange de ceux-ci.

9. Composition selon l'une quelconque des revendications précédentes, dans laquelle la quantité totale d'agent d'adhérence à la surface dentaire est de 10 % à 40 % en poids de la composition.

10. Composition selon l'une quelconque des revendications précédentes, dans laquelle le constituant hydrophobe comprend en outre un hydrocarbure naturel ou synthétique, par exemple de l'huile minérale, de la vaseline ou de la paraffine liquide, par exemple de la vaseline blanche, ou
dans laquelle la composition ne comprend pas d'agent antimicrobien ou de conservateur, par exemple, ne comprend pas de chlorure de cétylpyridinium, ou d'oxyde de zinc, ou les deux.

11. Composition selon l'une quelconque des revendications précédentes, dans laquelle la composition comprend :
| **Ingrédient** | **% en poids** |
|---|---|
| Adhésif silicone | 20 à 40 (p. ex. 30) |
| Fluide silicone | 10 à 40 (p. ex. 25) |
| Complexe PVP-H₂O₂ (p. ex. 18 % en poids de H₂O₂) | 15 à 35 (p. ex. 25) |
| STPP | 0,05 à 3 (p. ex. 0,1) |
| Polymère hydrophobe (p. ex. vaseline blanche) | 10 à 30 (p. ex. 18) |
| Aromatisant, édulcorant | 0,5 à 3 (p. ex. 1,1) |
| Silice | 0 à 5 (p. ex. 0,5 ou 1,0) |
ou
| **Ingrédient** | **% en poids** |
|---|---|
| Adhésif silicone | 20 à 40 (p. ex. 30) |
| Fluide silicone | 10 à 30 (p. ex. 18) |
| Complexe PVP-H₂O₂ (p. ex. 18 % en poids de H₂O₂) | 0,05 à 5,5 (p. ex. 0,55) |
| STPP | 0,05 à 3 (p. ex. 2) |
| Polymère hydrophobe (p. ex. vaseline blanche) | 10 à 30 (p. ex. 25) |
| Agent d'adhérence (p. ex. PVP ou cPVP) | 10 à 30 (p. ex. 22) |
| Aromatisant, édulcorant | 0,5 à 3 (p. ex. 1,1) |
| Silice abrasive | 0 à 5 (p. ex. 1,0) |

12. Composition selon l'une quelconque des revendications précédentes pour une utilisation dans un procédé de blanchiment d'une dent comprenant l'application de ladite composition sur une dent d'un mammifère.

13. Composition pour une utilisation selon la revendication 12, dans laquelle la composition est appliquée à l'aide d'un stylo.

14. Composition pour une utilisation selon la revendication 12 ou 13, dans laquelle le stylo est stocké dans un instrument de soins bucco-dentaires.
